# EUROPEAN PATENT APPLICATION

(11) **EP 1 132 445 A2**
(43) Date of publication of application: **12.09.2001**
(21) Application number: 01301852.8
(22) Date of filing: 28.02.2001
(51) Int. Cl.: C09J 11/04

(54) **Construction material**

(30) Priority: 01.03.2000 JP 2000055398
(71) Applicant: Motai, Takeji, Chofu-shi, Tokyo (JP); Fujii, Toshiaki, Sagamihara-shi, Kanagawa-ken (JP)
(72) Inventor: Motai, Takeji, Chofu-shi, Tokyo (JP); Fujii, Toshiaki, Sagamihara-shi, Kanagawa-ken (JP)
(74) Representative: Stanley, David William

(57) **Abstract**

A method of producing a health-promoting construction material includes processing a construction material by use of an adhesive (10a) to which a finely pulverized powder (11) of stone generating far-infrared radiation and/or negative ions has been incorporated. The stone (11) generating far-infrared radiation and/or negative ions is preferably quartz porphyry, granite porphyry, tourmaline, zeolite, or a mixture thereof.

## Description

The present invention relates to construction materials (the term refers to a material used in the construction of buildings and components of buildings such as ceilings, walls, and floors) for interior finishing of rooms where people live, work, or eat; e.g., rooms in a house or in an office, dining room, and similar places.

Conventionally, a working process for producing construction materials such as plywood, laminated veneer lumber, finished plywood, adhesive-laminated lumber, and particleboard generally employs adhesives comprising a formalin agent and resins such as urea resin, melamine resin, phenolic resin, and resorcinol resin. A formalin-like foul, irritating odor is conspicuously generated from such adhesives.

Accordingly, persons in the industry have been making efforts to reduce the level of formalin odor. However, the level has not yet been reduced to the standard level of formaldehyde in air inside a room - 0.08 ppm, as defined by WHO (World Health Organization) and the Japanese Ministry of Health and Welfare (June 1997).

In addition, vinyl chloride resin sheets, which are most widely employed as an interior material, generate a foul, irritating odor originating from anti-fungal agents and preservatives contained in plasticizers and adhesives employed in the sheets and from organic solvents; e.g., toluene and xylene, employed in paints and transparent coatings. Such an odor has an unfavorable impact on our lives.

Recently, people have had their health affected or have complained of feeling ill as a result of foul, irritating odors generated from construction materials employed as interior materials, and such cases, which are recognized as social pollution issues, have increased in number. In many cases, the pollution issues are caused by formalin serving as a component of an adhesive employed in the construction material; a foul, irritating odor originating from plasticizers and adhesives containing an anti-fungal agent and a preservative contained in vinyl chloride resin sheets serving as interior decorative sheets; and a foul, irritating odor originating from toluene and xylene employed in paints.

Since late 2000, in Japan, the labeling of the performance of a construction material and designation of the places where the material is to be used has become mandatory when houses are constructed. Thus, solving the aforementioned problems is essential for people involved in the industry.

In view of the foregoing, the present inventors have conducted extensive studies, and have found that blending a finely pulverized powder of stone such as quartz porphyry into an adhesive can have the effect of purifying the air in the room by absorbing and decomposing harmful matter such as fumes, dust, or sources of odor, as well as radiating far-infrared rays and so-called "negative ions" (negatively charged particles) into the room, to thereby maintain and promote blood flow in the human body. Preferred embodiments of the present invention have been accomplished on the basis of this novel and highly socially oriented technique so as to support good health, or promote health, rather than creating pollution problems.

More generally, according to one aspect of the present invention, there is provided a method of producing a construction material, including the step of applying to a component of the construction material an adhesive which incorporates a finely pulverized powder of stone generating far-infrared radiation and/or negative ions.

Such a method may include the step of bonding a decorative construction material to a construction base material by use of said adhesive.

The decorative construction material may be a decorative veneer plate or a decorative sheet.

A method as above may include the step of applying a coating material to a surface of the construction material, wherein said adhesive is admixed with said coating material.

Preferably, the construction material is selected from among plywood, laminated veneer lumber, adhesive-laminated lumber, particleboard, and medium density fiber board (MDF).

A method as above may include the step of bonding a decorative construction material to an interior base material by use of said adhesive.

The decorative construction material may be a decorative veneer plate or a decorative sheet.

A method as above may include the step of applying a coating material to an interior base material, wherein said adhesive is admixed with said coating material.

Preferably, the interior base material is selected from among floor material, wall material, and ceiling material.

Preferably, in any of the above methods, the finely pulverized powder of stone generating far-infrared radiation and/or negative ions to be incorporated into the adhesive is formed from raw stone selected from the group consisting of quartz porphyry, granite porphyry, tourmaline, zeolite, and a mixture thereof.

Preferably, said adhesive is an isocyanate-resin-based adhesive.

Constructions materials produced in accordance with embodiments of the invention may have health-promoting properties, and are referred to below as health-promoting construction materials, or "HC" materials.

For a better understanding of the invention, and to show how embodiments of the same may be carried into effect, reference will now be made, by way of example, to the accompanying diagrammatic drawings, in which:
FIG. 1A shows an adhesive to be mixed with a stone powder employed in Example 1 according to on e example of a method of embodying the present invention, for producing an HC material;
FIG. 1B shows the stone powder employed in Example 1 for producing an HC material;
FIG. 1C shows a stirrer for mixing the adhesive with the stone powder employed in Example 1 for producing an HC material;
FIG. 1D shows a step of applying the adhesive to a unit plate of laminated veneer lumber for producing an HC material in Example 1;
FIG. 1E shows a laminate to be pressed, employed in Example 1, for producing an HC material;
FIG. 1F shows a press machine for pressing the laminate employed in Example 1, for producing an HC material;
FIG. 1G shows laminated veneer lumber produced in Example 1;
FIG. 2A shows the laminate of FIG. 1G and a piece cut therefrom, along the grains;
FIG. 2B shows the laminate of FIG. 1G and a piece cut therefrom, in a direction perpendicular to that shown in FIG. 2A;
FIG. 3A shows a base plywood plate employed in Example 2 according to an example of the present invention, for producing an HC material;
FIG. 3B shows a decorative veneer plate employed in Example 2 according to an example of the present invention, for producing an HC material; and
FIG. 3C shows a hot press producing decorative laminated veneer lumber employed in Example 2, for producing an HC material.

Embodiments for carrying out methods of producing an HC material according to examples of the present invention will now be described.

In a first embodiment, during the production of construction materials such as plywood, laminated veneer lumber, and adhesive-laminated lumber, finely pulverized powder of quartz porphyry is uniformly mixed with an adhesive, in an amount of 5-30 wt.% depending upon application, and the adhesive is applied onto a surface to be processed in a coating amount of 150-300 g/m². When the target construction material is plywood, the thus-prepared unit plates are pressed at 10-15 kg/cm² at 110-120°C by use of a hot press, whereas when the target is laminated veneer lumber or adhesive-laminated lumber, the unit plates are pressed at 10-15 kg/m² by use of a cold press. Processing conditions such as pressure, temperature, and time are modified in accordance with the type and coating thickness of the adhesive, the number of unit plates to be pressed, and other conditions, and the processing can be performed under conditions known by those having ordinary skill in the art.

The produced laminated veneer lumber is cut from a top surface in such a manner as to provide side face pieces or end grain face pieces of predetermined thickness. It has already been proven that, through use of such pieces having a cross-section exposed to air, the effects of far-infrared radiation and/or negative ions exerted on room air are enhanced. In addition, a foul, irritating odor generated from the adhesive can be adsorbed and decomposed by incorporating a finely pulverized powder of stone generating far-infrared radiation and/or negative ions. Physical analysis through X-ray diffractometry has revealed that quartz porphyry and granite porphyry are porous minerals having a specific surface area of 200 m²/g or more and comprise 25,000 or more species of components.

The mechanism of the adsorption includes physical adsorption and chemical adsorption. Substances in air are trapped in pores of the stone powder, and then, harmful components are selectively adsorbed through chemical adsorption. In addition, the two aforementioned mineral species exhibit electrochemical adsorption-decomposition performance due to polarity existing therein. The two minerals further accelerate chemical reaction through catalysis which is unique to porous minerals, thereby modifying components and pH of aqueous solution and eluting mineral components. On the basis of the aforementioned actions, these two minerals can purify air and promote health.

Since these stone powders - or mineral powders - generate far-infrared radiation and/or negative ions semi-permanently, the construction materials fabricated by use of the adhesive containing such stone powder exhibit the effects for a long period of time after application thereof.

In a second embodiment, particleboard and medium density fiber board (MDF) are processed through solidification of small wood pieces by use of an adhesive. The industry of producing such processed boards can therefore further be boosted, because wood pieces can be utilized effectively to solve environmental problems. However, due to the market demand for low prices, the industry is forced to use large amounts of inexpensive formalin-containing adhesives in the production of such processed boards. In order to remove formalin, porous, multicomponent, polarized minerals such as quartz porphyry and granite porphyry are useful, as they chemically adsorb and decompose formalin. In the second embodiment, powders of these minerals are incorporated into an adhesive, to thereby prevent environmental pollution.

In a third embodiment, an adhesive containing a finely pulverized powder of stone such as quartz porphyry or granite porphyry is applied onto a surface of a construction material such as plywood, laminated veneer lumber, adhesive-laminated lumber, or particleboard, and a plurality of the thus-prepared precursors are processed by use of a hot press or a cold press by a process known in the art, to thereby produce HC materials. In the third embodiment, harmful substances such as formalin which are generated from a certain type of adhesive are adsorbed and decomposed, and air purification and promotion of human health through far-infrared radiation and/or negative ions are envisaged.

In a fourth embodiment, a coating material is applied onto a surface of a construction material or an interior material such as wall material, ceiling material, or floor material. In some cases, a coating material generates a foul, irritating odor more intense than the odor generated from an adhesive. In order to remove such odor, a finely pulverized powder of a porous, multi-component, polarized mineral is incorporated into a coating material. The fourth embodiment is directed to such a feature.

Particularly, since a surface to which a coating material is applied greatly affects the air in a room and human health, the fourth embodiment has a strong impact on the improvement of human health.

In a fifth embodiment, a variety of minerals which generate far-infrared radiation and/or negative ions are employed to prepare a finely pulverized powder. A variety of candidates selected from specimens collected worldwide have been tested, and, as a result, the most effective minerals found are quartz porphyry and granite porphyry, which occur in Japan. Tourmaline and zeolite are supplemental minerals, in view of quality and price. These two minerals are of particularly high value when large amounts of such minerals are required, or when high-quality quartz porphyry or granite porphyry is unavailable.

In a sixth embodiment, a finely pulverized powder of quartz porphyry is added to a coating material, and the mixture is homogeneously kneaded by use of a mixer. Then, the thus-prepared coating material is applied to a surface of a construction material which is to be installed in a room or has already been installed in a room.

Throughout these embodiments, when decorative unit plates are used, thin plates each having a thickness of approximately 0.3-0.5 mm are preferred, to thereby effectively diffuse, through the thin unit plates to a room, far-infrared radiation and/or negative ions generated from quartz porphyry contained in the adhesive.

After vinyl chloride resin sheets are laminated on a substrate, the entire structure is pressed by use of a cold press. During pressing, toxic substances or a plasticizer contained in the adhesive are decomposed to non-toxic species. In addition, the aforementioned effects of far-infrared radiation and/or negative ions are provided through the thin vinyl chloride resin sheets.

In examples of the present invention, adhesives that include formalin may be employed. However, formalin-free isocyanate-based adhesives, such as isocyanate polymer adhesives, are preferred in view of more effective prevention of odor.

Effects of far-infrared radiation and/or negative ions generated from stone-powder-containing HC material have already been recognized. That is, the far-infrared radiation and/or negative ions penetrate the human skin and permeate the human body to a depth of 4-5 cm, thereby exerting an effect on a number of cells contained in muscle layers, blood vessels, and lymphatic vessels. As a result, activation of blood circulation and lymphocytes is promoted, to thereby enhance immunity and maintain and promote human health.

The research on quartz porphyry conducted by Takeo ONO, a professor at Gifu Pharmaceutical University, is summarized in the following four points.
(1) Detection of α-ray and magnetic lines of force
   α-Ray and magnetic lines of force generated from quartz porphyry have been detected. These are considered to be generated from radioactive, colorless zircon contained in quartz porphyry and exert their effect on the human body. Quartz porphyry generates far-infrared radiation, which activates blood and cells in the human body and generates negative charges in air, to thereby form negative ions; i.e., negatively charged oxygen or carbon dioxide. The thus-formed negative ions are thought to provide good effect on the human body.
(2) Minerals having ultra-high porosity
   When quartz porphyry is pulverized into a powder of 500 mesh or more, a sponge-like, highly porous texture is observed under an electron microscope. Specific surface area reaches some hundreds square meters per gram. The pores capture and physically adsorb harmful substances. In addition, quartz porphyry *per se* has polarity, which also provides electric adsorption.
(3) Minerals containing multiple components
   Through powder X-ray diffractometry, quartz porphyry has been found to contain 25,000 or more components. Of such numerous components, silicic acid is a predominant component, and other components are contained in a well-balanced manner for our purposes. As a result, quartz porphyry adsorbs harmful substances not only physically but also chemically (to thereby decompose the substances). This mechanism prevents saturation of adsorption of the harmful substances.
(4) Modification of clusters of water and pH
   In water, H₂O molecules do not exist as isolated molecules, one from another, but form clusters through intermolecular hydrogen bonds. The smaller the size of a cluster, the better the taste of water. In addition to ion exchange action of magnesium ions, calcium ions, and similar ions, quartz porphyry can regulate the size of clusters to an appropriate size through action of α-rays in combination with electromagnetic waves, including far-infrared radiation. Quartz porphyry also exhibits a buffer action, adjusting pH of an aqueous solution.

Attempts have been made to numerically express these effects by measuring undulatory property ― which any substance possesses - through use of a mechanical means.

More specifically, any existing substance has an inherent oscillation number (due to nuclear vibration). The oscillation can be detected by means of a measuring apparatus and can be expressed as an undulatory index, which also serves as an index for far-infrared radiation. Accordingly, measurement of the undulatory index provides the level of effect of far-infrared radiation on human health; e.g., positive effects and negative effects.

Tables 1 and 2 show undulatory indices of naturally occurring minerals (from +20 to -20).

The numerical data in Tables 1 and 2 are obtained by "*Hado Ikagaku Sogo Kenkyu-sho*," which organization specializes in study of undulatory indices.

**Table 1**

| Undulatory indices of naturally occurring energy minerals | |
|---|---|
| Minerals | Undulatory Index |
| Quartz porphyry | +14 |
| Granite porphyry | +13 |
| Tourmaline | +12 |
| Bamboo charcoal (*Chiku-tan*) | +11 |
| Zeolite | +10 |
| Charcoal of Japanese oak (*Bin-cho tan*) | +9 |
| Titanium dioxide | +8 |
| Quartz | +7 |

**Table 2**

| Comparison of undulatory indices: *Hado-sui*, in which energy mineral micropowder (quartz porphyrite 44-µ powder) providing a high undulatory index is dissolved, vs. commercially available mineral water | | | | | |
|---|---|---|---|---|---|
| (by *Hado Ikagaku Sogo Kenkyu-sho*) | | | | | |
| Water | Immunity | Allergy | Atopy | Liver | Cancer |
| *Hado-sui* | +20 | +20 | +20 | +20 | +20 |
| *Misumino-ko sui* | +16 | +15 | +14 | +13 | +15 |
| *Yakusima Jyomon sui* | +5 | +5 | +4 | +5 | +5 |
| *Okuhida Tenro sui* | +5 | +6 | +5 | +4 | +7 |
| *Hikyo Kurobe simizu* | +6 | +3 | +5 | +4 | +5 |
| *Fuji mineral water* | +5 | +5 | +6 | +5 | +5 |
| *Kurama-tengu no mizu* | + 11 | +10 | +11 | +10 | +11 |
| Tap water in Nagoya city | ±0 | -0 | -0 | ±0 | ±0 |

The indices shown in Tables 1 and 2 fall within a range of +20 to -20. The relationship between the index and effect on human health is summarized in Table 3.

**Table 3**

| Undulatory index | Effects |
|---|---|
| ≥15 | Promoting health |
| ≥10 | Maintaining health |
| ≤0 | Possibly harmful to the human body |
| ≤-10 | Harmful to the human body (hazardous) |

The higher the index, the greater the "healing effect" to organs of the human body.

### EXAMPLES

### Example 1

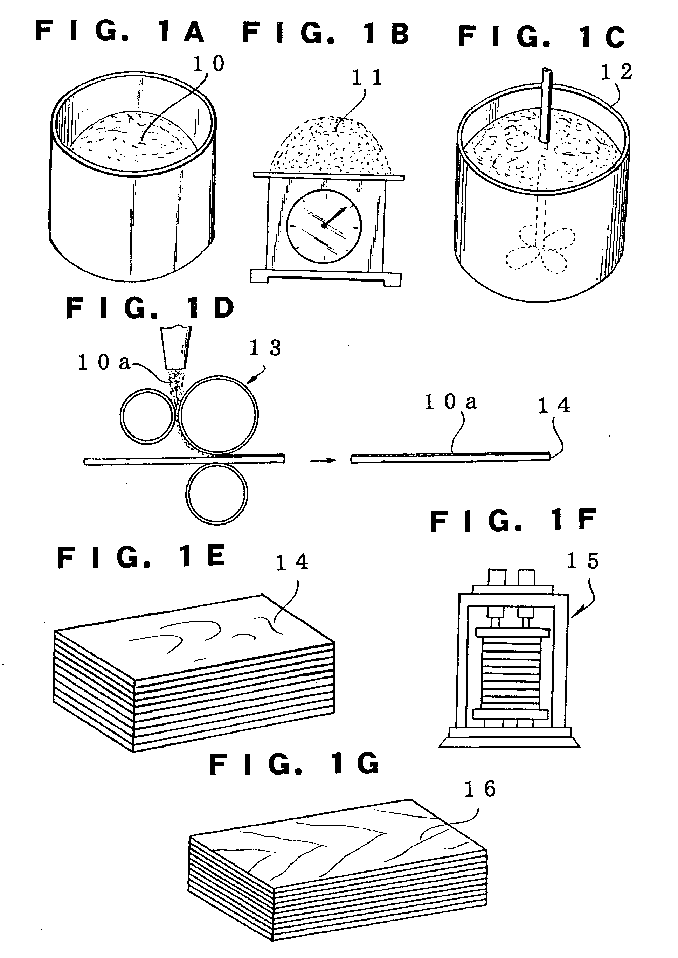

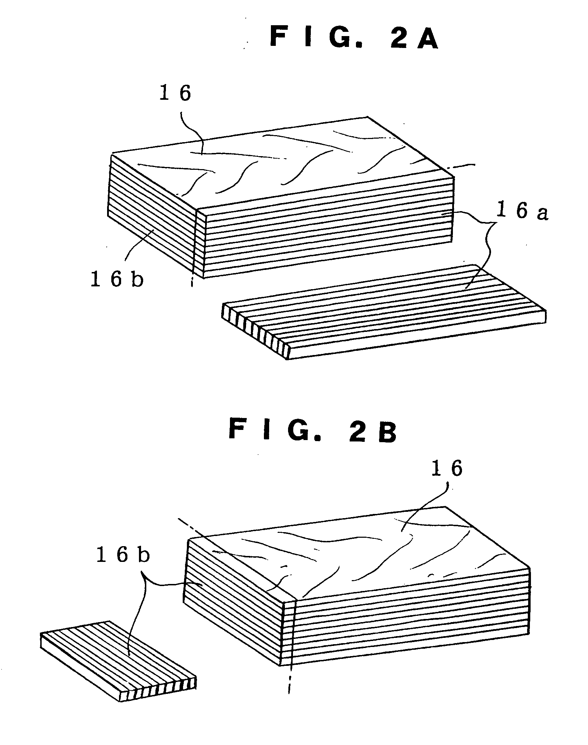

In Example 1, a method of producing laminated veneer lumber, which is one of the most important HC materials, will be described with reference to FIGs. 1 and 2.

In Example 1, quartz porphyry was pulverized into a micropowder, and the resultant micropowder was used for producing HC material. The advantages of using quartz porphyry in the production of HC materials are supported by the aforementioned research conducted by Takeo ONO, a professor at Gifu Pharmaceutical University.

The method of producing laminated veneer lumber is described with reference to FIGs. 1A to 1G. To an adhesive 10 shown in FIG. 1A, quartz porphyry 11 (50 µ micropowder) shown in FIG. 1B is added. As shown in FIG. 1C, the resultant mixture is stirred by means of an automatic stirrer 12 until a homogenous mixture is attained. As shown in FIG. 1D, the resultant quartzporphyry-containing adhesive 10a is uniformly applied to a laminated veneer lumber unit plate 14 by means of an adhesive-applying roll-spreader 13.

Subsequently, a plurality of the unit plates 14 coated with the adhesive 10a shown in FIG. 1D are stacked in parallel in the manner illustrated in FIG. 1E. Then, the formed laminate is pressed by means of a cold-press 15 shown in FIG. 1F, to thereby yield laminated veneer lumber 16 shown in FIG. 1G, for producing HC material.

In a subsequent step, the obtained laminated veneer lumber 16 shown in FIG. 1G is cut into pieces of a certain thickness in accordance with the mode of use. For example, as shown in FIG. 2A, the laminated veneer lumber 16 is cut from an upper surface, to thereby expose a side face 16a, or as shown in FIG. 2B, the laminated veneer lumber 16 is cut so as to expose a side surface 16b. These cut pieces are employed as HC materials; e.g., floor materials, wall materials, ceiling materials, and furniture materials.

Example 1 will be described in more detail.

To an aqueous vinyl urethane adhesive 10 (10 kg) containing no formalin generating a foul, irritating odor, a powder of finely pulverized quartz porphyry (50 µ, collected from Aichi Prefecture, Japan) 11 was added in an amount of 20 wt.%. The mixture was kneaded by means of an automated stirrer 12 in which stirring paddles rotate while the rotation axis continuously moves up and down, to thereby obtain a stone-powder-containing adhesive 10a. The adhesive was applied onto a surface of a unit plate 14 (3 mm thickness, 600 mm width, and 200 mm length) made of southern yellow pine, in a coating amount of 250 g/m². Although a plurality of unit plates 14 were coated in such a manner, no adhesive was applied to a surface of a unit plate to be placed on the top of a laminate.

Ten unit plates 14 were stacked in a parallel manner, and the formed laminate was pressed by means of a cold press 15 at 13 kg/cm² for 30 minutes, to thereby obtain a block of laminated veneer lumber 16 (30 mm thickness, 600 mm width, and 200 mm length). In a similar manner, ten blocks of the laminated veneer lumber were prepared. To a surface of each block, a stone-powder-containing adhesive 10a ― the same adhesive that had been applied to unit plates 14 - was applied in a coating amount of 300 g/m². Ten blocks were stacked and pressed in a manner similar to that described above by means of a cold press 15 at 15 kg/cm² for 20 minutes, to thereby obtain a large block of laminated veneer lumber 16 (300 mm thickness, 600 mm width, and 200 mm length).

The large block was cut in a direction normal to the 300-mm-thick plane, to provide a plate piece 16a (10 mm thickness, 300 mm width, and 200 mm length). A 300-mm-thick plane thereof was employed as a wall material. In the material, nine adhesive layers are exposed to the air. Thus, far-infrared radiation and/or negative ions are semi-permanently generated from the adhesive layers. When a wall is fabricated from the material, considerably large amounts of far-infrared radiation and/or negative ions are provided.

On the basis of the aforementioned analyses of quartz porphyry conducted by Takeo ONO, a professor at Gifu Pharmaceutical University, the present inventors attempted to confirm favorable properties of the HC material produced in Example 1 by use of an adhesive to which a finely pulverized powder of quartz porphyry had been incorporated. To this end, the present inventors had a research center, *Hado Ikagaku Sogo Kenkyu-sho*, test the properties on the basis of the standards established by that organization. The results are shown in Table 4.

**Table 4**

| Test items | LVL^{*} produced by use of adhesive containing quartz porphyry | LVL^{*} produced by use of adhesive containing no quartz porphyry |
|---|---|---|
| Undulatory index | 15 | 9 |
| Far-IR ray | 14 | 9 |
| Negative ions | 15 | 8 |
| Immunity | 14 | 7 |
| Stress (healing effect) | 14 | 7 |

| | | |
|---|---|---|
| ^{*} LVL: laminated veneer lumber | | |

### Example 2

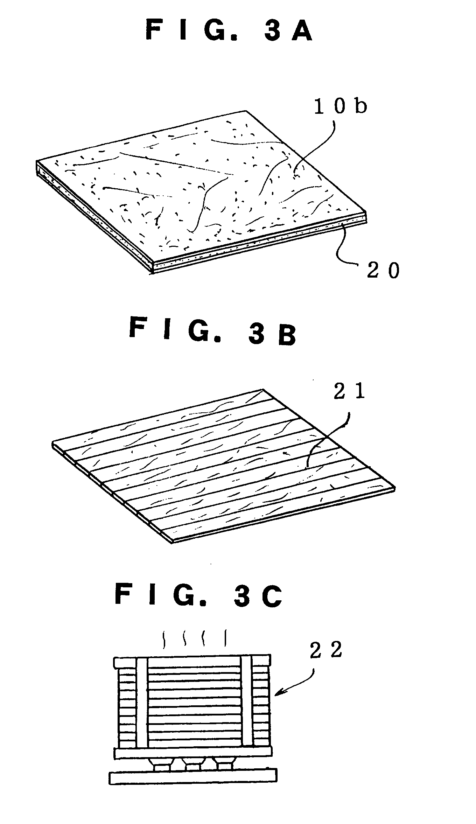

With reference to FIGs. 3A to 3C, steps for producing decorative laminated veneer lumber will be described. A stone-powder-containing adhesive 10a-employed in Example 1 as shown in FIG. 1C ― was applied onto a base plywood plate 20 as shown in FIG. 3A. A decorative veneer plate 21 as shown in FIG. 3B was stacked on the adhesive-coated base plywood plate, and a plurality of the resultant laminates were pressed under heat by means of a hot press 22 as shown in FIG. 3C, to thereby provide an HC material.

More specifically, a micropowder of *Bakuhan-seki* (granite porphyry) (50 µ) was added in an amount of 20 wt.% to aqueous vinyl urethane, to thereby prepare a stone-powder-containing adhesive 10b. The adhesive was applied onto the base plate 20 in a coating amount of 150 g/m². Each of two decorative veneer plates (0.3 mm and 1 mm thickness) was stacked on the adhesive-coated base plywood plate, and a plurality of the resultant laminates were pressed under heat by means of the hot press 22, to thereby provide an HC material. A reference material was also prepared in a similar manner, except that a decorative veneer plate (1 mm) and an adhesive containing no stone powder were used. The performances of the thus-obtained HC materials are shown in Table 5. As is clear from Table 5, the performance of HC materials of examples of the present invention is far superior to that of the reference material.

**Table 5**

| Test items | Base plate (4 mm) Decorative veneer (0.3 mm) Granite porphyry powder (20%) | Base plate (4 mm) Decorative veneer (1 mm) Granite porphyry powder (20%) | Base plate (4 mm) Decorative veneer (1 mm) Granite porphyry powder (none) |
|---|---|---|---|
| Undulatory index | 14 | 10 | 9 |
| Far-IR ray | 12 | 9 | 9 |
| Negative ions | 12 | 9 | 8 |
| Immunity | 11 | 8 | 7 |
| Stress (healing effect) | 13 | 8 | 8 |

As described hereinabove, in the methods of producing an HC material according to examples of the present invention, an adhesive to which a finely pulverized powder of stone such as quartz porphyry or granite porphyry generating far-infrared radiation and/or negative ions has been incorporated in an appropriate amount is employed during production of construction materials such as laminated veneer lumber and decorative laminated veneer lumber. The thusproduced HC materials have been proven to possess positive health effects as compared with similar materials produced by use of an adhesive containing no such stone powder.

Finely pulverized powder of stone such as quartz porphyry or granite porphyry generates far-infrared radiation and/or negative ions semi-permanently. Therefore, when an HC material containing the stone powder is used, the effect due to stone such as quartz porphyry or granite porphyry will last for a long period of time. In addition, quartz porphyry and granite porphyry are characterized by having a specific surface area (porosity) of some hundreds of square meters per gram; by including approximately 25,000 components (multi-chemical components); and by being endowed with polarity and an α-ray-generating property (electric properties). Thus, harmful substances such as fumes and dust in a room are adsorbed and decomposed, to thereby purify the air in the room.

In examples of the present invention, in place of quartz porphyry or granite porphyry, powders of minerals such as zeolite or tourmaline may be employed as the finely pulverized mineral powder of stone to be incorporated into an adhesive which is employed during processing to produce the aforementioned laminated veneer lumber or decorative laminated veneer lumber. However, the effects due to the minerals may be weaker to a certain degree.

Also, in the case in which an adhesive containing an appropriate amount of a finely pulverized powder of quartz porphyry is applied to a surface of laminated veneer lumber or plywood, and a decorative sheet such as decorative paper is laminated onto the adhesive-coated substrate, quartz porphyry contained in the adhesive generates far-infrared radiation and/or negative ions which penetrate the decorative sheet, to provide the aforementioned effects to air in a room.

When the decorative sheet is a vinyl chloride resin sheet, harmful substances contained in an agar-based adhesive can be decomposed by the stone powder, and the aforementioned effects are provided through such a thin decorative sheet to air in the room.

Furthermore, the effects of examples of the present invention can also be provided when a coating material containing a finely pulverized powder of stone such as quartz porphyry is applied onto a surface of a construction material such as laminated veneer lumber, plywood, or plaster board so as to produce a coated construction material.

In relation to the aforementioned methods, a variety of minerals which generate far-infrared radiation and/or negative ions may be incorporated into an adhesive or a coating material and tested in terms of performance, price, safety in supply, and operability. A variety of candidates selected from specimens collected worldwide have been tested, and as a result, it has been found that quartz porphyry, granite porphyry, tourmaline, and zeolite are advantageously employable. In particular, of these mineral species, quartz porphyry and granite porphyry have been found to be most preferable in the plywood industry and the particleboard industry.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, abstract and drawings), or to any novel one, or any novel combination, of the steps of any method or process so disclosed.

## Claims

1. A method of producing a construction material (16), including the step of applying to a component (14) of the construction material (16) an adhesive (10a) which incorporates a finely pulverized powder (11) of stone generating far-infrared radiation and/or negative ions.

2. A method according to claim 1, including the step of bonding a decorative construction material (21) to a construction base material by use of said adhesive.

3. A method according to claim 2, wherein said decorative construction material (21) is a decorative veneer plate.

4. A method according to claim 2, wherein said decorative construction material (21) is a decorative sheet.

5. A method according to claim 1, including the step of applying a coating material to a surface of the construction material, wherein said adhesive (10a) is admixed with said coating material.

6. A method according to any of claims 1 to 5, wherein the construction material is selected from among plywood, laminated veneer lumber, adhesive-laminated lumber, particleboard, and medium density fiber board (MDF).

7. A method according to claim 1, including the step of bonding a decorative construction material (21) to an interior base material by use of said adhesive.

8. A method according to claim 7, wherein said decorative construction material (21) is a decorative veneer plate.

9. A method according to claim 7, wherein said decorative construction material (21) is a decorative sheet.

10. A method according to claim 1, including the step of applying a coating material to an interior base material, wherein said adhesive (10a) is admixed with said coating material.

11. A method according to any of claims 7 to 10, wherein the interior base material is selected from among floor material, wall material, and ceiling material.

12. A method according to any of the preceding claims, wherein the finely pulverized powder of stone generating far-infrared radiation and/or negative ions (11) to be incorporated into the adhesive is formed from raw stone selected from the group consisting of quartz porphyry, granite porphyry, tourmaline, zeolite, and a mixture thereof.

13. A method according to any of the preceding claims, wherein said adhesive (10) is an isocyanate-resin-based adhesive.
